# EUROPÄISCHE PATENTSCHRIFT

(11) **EP 2 962 653 B1**
(45) Veröffentlichungstag und Bekanntmachung des Hinweises auf die Patenterteilung: **20.09.2017**
(21) Anmeldenummer: 14175072.9
(22) Anmeldetag: 30.06.2014
(51) Int. Cl.: A61B 17/86, A61B 17/80

(54) **Knochenschraube**
Bone screw
Vis à os

(43) Veröffentlichungstag der Anmeldung: 06.01.2016
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll

(56) Entgegenhaltungen:
- WO-A1-97/09000
- WO-A2-2011/154891
- US-A1- 2010 057 086
- US-A1- 2014 018 862

## Beschreibung

Die Erfindung betrifft eine Knochenschraube zur Fixierung eines Verbindungsträgers an einem Knochen. Die Knochenschraube umfasst einen Schraubenkopf, welcher ein Kopfgewinde zur Herstellung einer Gewindeverbindung mit dem Verbindungsträger aufweist. Zudem umfasst die Knochenschraube einen Schaft, welcher ein Knochengewinde zum Eingriff in das Knochenmaterial aufweist. Zwischen dem Schraubenkopf und dem Schaft weist die Knochenschraube einen Übergangsbereich auf. Die Knochenschraube weist außerdem an ihrer Oberfläche eine harte Beschichtung auf.

Es ist bekannt Knochenschrauben zu verwenden, um einen Verbindungsträger, wie beispielsweise eine Knochenplatte, an einen Knochen zu fixieren. Verwendung finden Knochenschrauben beispielsweise bei der Behandlung von Knochenfrakturen. Die Knochenschrauben werden durch Durchgangslöcher in der Knochenplatte geführt und in die Teilstücke des gebrochenen Knochens eingeschraubt. Das Knochengewinde greift dabei in das Knochenmaterial ein, so dass eine feste Verbindung zwischen Knochenschraube und Knochen entsteht. Zudem stellt das Kopfgewinde eine feste Gewindeverbindung mit der Knochenplatte her. Die Fraktur wird auf diese Weise von der Knochenplatte überbrückt, so dass die Teilstücke stabilisiert werden und wieder zusammenwachsen können. Knochenschrauben können auch zusammen mit anderen Arten von Verbindungsträgern, wie beispielsweise Elementen von Gelenkprothesen, verwendet werden.

Es ist weiterhin bekannt, Knochenschrauben mit einer harten Beschichtung zu versehen. Harte Beschichtungen dienen dazu, die mechanische Belastbarkeit zu erhöhen und beispielsweise einen Abrieb des Materials der Knochenschraube während des Einschraubens und eine damit verbundene Partikelbildung zu verringern oder zu verhindern. Abgeriebene Partikel sind unerwünscht, da sie innerhalb des Knochengewebes Fremdkörper darstellen und zu unerwünschten Reaktionen führen können. Eine Knochenschraube mit einer eloxierten Beschichtung ist aus US 2010/0057086 A1 bekannt.

Die Schichtdicke der Beschichtung ist üblicherweise so bemessen, dass sie trotz der beim Eindrehen der Knochenschraube auftretenden Belastungen einen unmittelbaren Kontakt zwischen dem beschichteten Material der Knochenschraube und dem umgebenden Material verhindert. Insbesondere kann im Bereich des Schafts ein Kontakt zwischen dem beschichteten Material und dem Knochengewebe vermieden werden. Dies ist erwünscht, da das Kernmaterial durch im Knochenmaterial vorhandene Aminosäuren angegriffen werden kann. Allerdings haben solche harten Beschichtungen den Nachteil, dass sie nicht förderlich für die Festigkeit der Gewindeverbindung zwischen dem Schraubenkopf und dem Verbindungsträger sind.

Davon ausgehend ist es die Aufgabe der vorliegenden Erfindung eine Knochenschraube vorzustellen, welche eine verbesserte Verbindungsfestigkeit zwischen dem Kopf der Knochenschraube und dem Verbindungsträger aufweist. Diese Aufgabe wird gelöst mit den Merkmalen der unabhängigen Ansprüche. Vorteilhafte Ausführungsformen finden sich in den Unteransprüchen.

Erfindungsgemäß weist die Beschichtung im Bereich des Schraubenkopfes eine geringere Dicke auf als im Bereich des Schafts.

Zunächst werden einige im Rahmen der Erfindung verwendete Begriffe erläutert. Eine Beschichtung wird als hart bezeichnet, wenn sie eine größere Härte als das beschichtete Material aufweist. Bei der Ermittlung relativer Härten führen die unterschiedlichen Härte-Messverfahren regelmäßig zu identischen Ergebnissen. Beispielsweise kann die Härte nach Rockwell ermittelt werden (DIN EN ISO 6508-1).

Ein Übergangsbereich der Knochenschraube bezeichnet einen Teil der Schraube, welcher in Längsrichtung der Schraube zwischen dem Schraubenkopf und dem Schaft angeordnet ist. Im Übergangsbereich ist die Schichtdicke der Beschichtung nicht festgelegt. Beispielsweise kann die Schichtdicke am schaftseitigen Ende des Übergangsbereiches mit der Schichtdicke am Schaft übereinstimmen. Am kopfseitigen Ende des Übergangsbereiches kann die Schichtdicke mit der Schichtdicke am Schraubenkopf übereinstimmen. Zwischen dem schaftseitigen und dem kopfseitigen Ende kann die Schichtdicke beliebig variieren, beispielsweise kontinuierlich abnehmen.

Die Erfindung beruht auf der Erkenntnis, dass eine harte Beschichtung mit einer großen Dicke im Bereich des Schraubenkopfes nachteilig sein kann und die Verbindungsfestigkeit zwischen dem Kopfgewinde und dem Verbindungsträger beeinträchtigen kann. Dies gilt insbesondere dann, wenn Knochenschraube und Verbindungsträger aus einem gleichartigen Material, beispielsweise aus Titanlegierungen oder aus Reintitan bestehen. Die harte Beschichtung verhindert in diesem Fall nämlich, dass die gleichartigen Materialien in unmittelbaren Kontakt kommen. Die Erfindung hat erkannt, dass ein unmittelbarer Kontakt zwischen Schraubenkopf und Verbindungsträger aber vorteilhaft ist, da aufgrund einer chemischen Verbindung zwischen den gleichartigen Materialien die Verbindungsfestigkeit erhöht wird. Indem die Erfindung nun vorschlägt, am Schraubenkopf eine dünnere Beschichtung vorzusehen, wird beim Herstellen der Gewindeverbindung zwischen Schraubenkopf und Verbindungsträger erreicht, dass Schraubenkopf und Verbindungsträger zumindest teilweise in unmittelbarem Kontakt stehen und eine feste Verbindung miteinander eingehen können. Trotzdem ist die Beschichtung noch so dick, dass sie in den zu der Gewindeverbindung benachbarten Bereichen einen freien Kontakt des beschichteten Materials zu dem umgebenden Gewebe verhindert.

Das erfindungsgemäße Kopfgewinde zur Herstellung einer Gewindeverbindung mit dem Verbindungsträger kann als Gewinde zum Eingriff in ein vorhandenes Gegengewinde des Verbindungsträgers ausgestaltet sein. Das Kopfgewinde kann auch als selbstschneidendes Gewinde ausgestaltet sein, welches das Material des Verbindungsträgers beim Eindrehen umformt, um die Gewindeverbindung herzustellen.

In einer vorteilhaften Ausführungsform weist die Beschichtung im Bereich des Schraubenkopfes eine Dicke zwischen 10 nm und 70 nm, bevorzugt zwischen 20 nm und 50 nm auf. Eine so gewählte Beschichtungsdicke im Bereich des Schraubenkopfes führt dazu, dass die gleichartigen Materialien des Schraubenkopfes und des Verbindungsträgers unmittelbar aufeinander zu liegen kommen. Es kommt so zu einer festen Verbindung zwischen Schraubenkopf und Verbindungsträger.

Die Beschichtung kann im Bereich des Schafts eine Dicke zwischen 80 nm und 5 µm haben. Die Herstellung ist einfacher, wenn die Schichtdicke am Schaft nur geringfügig größer ist als am Schraubenkopf und beispielsweise zwischen 80 nm und 200 nm, bevorzugt zwischen 90 nm und 120 nm, weiter bevorzugt bei etwa 100 nm liegt. Um das beschichtete Material zuverlässig von der Umgebung zu trennen, ist eine Schichtdicke am oberen Ende des beanspruchten Bereichs von Vorteil, beispielsweise zwischen 1 µm und 5 µm. Durch die im Bereich des Schafts größere Schichtdicke hält die Schraube den beim Eindrehen der Schraube auftretenden Belastungen stand. Das beschichtete Material am Schaft der Knochenschraube bleibt auf diese Weise durch die Beschichtung vor unmittelbarem Kontakt mit dem Knochenmaterial geschützt.

In einer vorteilhaften Ausführungsform umfasst die Beschichtung der Knochenschraube Titanoxid. Der Begriff Titanoxid umfasst TiO₂, aber auch die Suboxide des Titans wie beispielsweise Ti₂O₃ oder Ti₃O₅. Die Beschichtung kann beispielsweise durch eine anodische Oxidation der Knochenschraube hergestellt werden. Mit dieser Methode kann auf einfache Weise eine harte Beschichtung erzeugt werden. Das Titanoxid bildet vorzugsweise den Hauptbestandteil der Beschichtung. Die Beschichtung der Knochenschraube kann auch vollständig aus Titanoxid bestehen.

In einer bevorzugten Ausführungsform besteht die Knochenschraube aus Reintitan, weiter bevorzugt aus Reintitan Grade 4. Die Knochenschraube kann auch aus einer Titanlegierung, bevorzugt aus TiAl6V4 bestehen.

Gegenstand der vorliegenden Erfindung ist zudem ein Fixationssystem für Knochen mit einem Verbindungsträger, welcher wenigstens ein Durchgangsloch aufweist, und mit wenigstens einer erfindungsgemäßen Knochenschraube. Die Knochenschraube kann in das Durchgangsloch zur Herstellung einer Gewindeverbindung zwischen dem Kopfgewinde und dem Verbindungsträger eingesetzt werden. Bevorzugt ist der Verbindungsträger aus Reintitan oder einer Titanlegierung. Dies bedeutet, dass das Reintitan bzw. die Titanlegierung den Hauptanteil des Verbindungsträgers bildet. Beispielsweise kann der Verbindungsträger abgesehen von einer Beschichtung vollständig aus Reintitan bzw. der Titanlegierung bestehen. Die Titanlegierung kann beispielsweise TiAl6V4 sein.

Der Verbindungsträger kann im Bereich eines Durchgangsloches ein Gegengewinde für das Kopfgewinde der Knochenschraube aufweisen.

In einer bevorzugten Ausführungsform ist der Verbindungsträger im Bereich eines Durchgangsloches aber dazu ausgestaltet, beim Herstellen einer Gewindeverbindung vom Kopfgewinde der Knochenschraube umgeformt zu werden. Bevorzugt besteht der Verbindungsträger dazu im Bereich eines Durchgangsloches aus einem Material, das weicher ist als das Material des Knochengewindes. Auf diese Weise kann der Winkel, den die Knochenschraube nach dem Herstellen der Gewindeverbindung relativ zum Verbindungsträger einnimmt, innerhalb eines Winkelbereichs frei gewählt werden. Es kann auf diese Weise eine multidirektional winkelstabile Verbindung zwischen Knochenschraube und Verbindungsträger hergestellt werden.

In einer vorteilhaften Ausführungsform umfasst der Verbindungsträger im Bereich eines Durchgangsloches eine Materiallippe, welche beim Herstellen einer Gewindeverbindung vom Kopfgewinde der Knochenschraube umgeformt wird. Die Materiallippe kann durch einen umlaufenden Vorsprung an der Innenseite eines Durchgangslochs gebildet sein. Bevorzugt besteht die Materiallippe aus einem weicheren Material als der übrige Teil des Verbindungsträgers.

Die Erfindung wird nachfolgend unter Bezugnahme auf die beigefügte Zeichnungen erläutert, in denen ein vorteilhaftes Ausführungsbeispiel dargestellt ist. Es zeigen:
- Figur 1:: eine Seitenansicht einer erfindungsgemäßen Knochenschraube;
- Figur 2:: einen Teilausschnitt der Knochenschraube aus Figur 1 in einer seitlichen Schnittansicht vor dem Einschrauben der Knochenschraube in einen Knochen bzw. einen Verbindungsträger;
- Figur 3:: einen Teilausschnitt der Knochenschraube aus Figur 1 in einer seitlichen Schnittansicht nach dem Einschrauben der Knochenschraube in den Knochen bzw. in den Verbindungsträger;
- Figur 4:: eine weitere Ausführungsform einer erfindungsgemäßen Knochenplatte in einer seitlichen Schnittansicht;
- Figur 5:: ein erfindungsgemäßes Fixationssystem in einer seitlichen Schnittansicht.

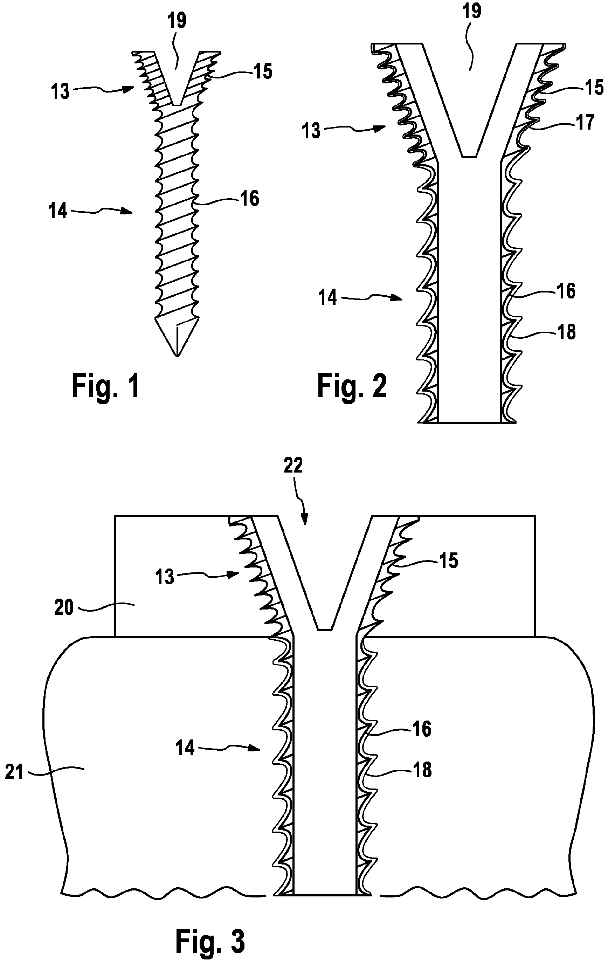

Figur 1 zeigt eine seitliche Ansicht einer Knochenschraube. Die Knochenschraube weist an ihrem oberen Ende einen Schraubenkopf 13 und unterhalb des Schraubenkopfes 13 einen Schaft 14 auf. Der Durchmesser des Schraubenkopfes 13 gleicht an seinem unteren Ende im Wesentlichen dem Durchmesser des Schafts 14. In Richtung des oberen Endes nimmt der Durchmesser des Schraubenkopfes 13 zu. In den Schraubenkopf 13 ist ein Werkzeugangriff 19 eingelassen, über den die Knochenschraube mit Hilfe eines geeigneten Werkzeugs gedreht werden kann.

Am Schraubenkopf 13 ist ein Kopfgewinde 15 angeordnet. Am Schaft 14 befindet sich zudem ein Knochengewinde 16. Die Knochenschraube besteht aus der Titanlegierung TiAl6V4, wobei die Knochenschraube durch eine anodische Oxidation mit einer Titanoxidschicht versehen wurde. Die Titanoxidschicht ist in Figur 2 gezeigt.

Figur 2 zeigt einen Teilausschnitt der Knochenschraube der Figur 1 in einer seitlichen Schnittansicht. Der Schnitt verläuft dabei durch die Längsachse der Knochenschraube. Figur 2 zeigt die Knochenschraube und insbesondere die Beschichtung der Knochenschraube in einem Zustand vor dem Einschrauben in einen Knochen bzw. in eine Knochenplatte. Dabei ist zu Illustrationszwecken die Dicke der Beschichtung nicht maßstabsgetreu, sondern deutlich vergrößert eingezeichnet.

Im Bereich des Kopfgewindes 15 ist die Knochenschraube mit einer Titanoxidbeschichtung 17 versehen, welche eine Dicke von 30 nm aufweist. Im Bereich des Schafts 14 ist die Knochenschraube mit einer Titanoxidbeschichtung 18 versehen, welche eine Dicke von 100 nm aufweist. Aufgrund ihrer größeren Dicke ist die Beschichtung 18 deutlich reibungsbeständiger als die Beschichtung 17.

Der Zustand der Knochenschraube nach dem Einschrauben in einen Knochen 21 sowie in eine auf dem Knochen 21 auffliegende Knochenplatte 20 ist in Figur 3 illustriert. Die Knochenplatte 20 besteht aus einer Titanlegierung und weist ein Durchgangsloch 22 auf. Die Knochenschraube wurde durch das Durchgangsloch 22 hindurchgeführt und in den Kochen 21 eingeschraubt. Das Knochengewinde 16 wurde dabei in Eingriff mit dem Knochenmaterial des Knochens 21 gebracht, so dass eine feste Verbindung zwischen dem Schaft 14 und dem Knochen 21 entsteht. Zudem wurde das Kopfgewinde 15 in Eingriff mit der Knochenplatte 20 gebracht. Trotz der starken Reibung zwischen dem Knochenmaterial und dem Schaft 14 hat trennt die dickere Beschichtung 18 das beschichtete Material des Schafts 14 vom umgebenden Knochenmaterial. Im Gegensatz dazu gibt es im Bereich der dünnen Beschichtung 17 keine vollständige Trennung, so dass das Kopfgewinde 15 in unmittelbarem Kontakt mit der Knochenplatte 20 steht.

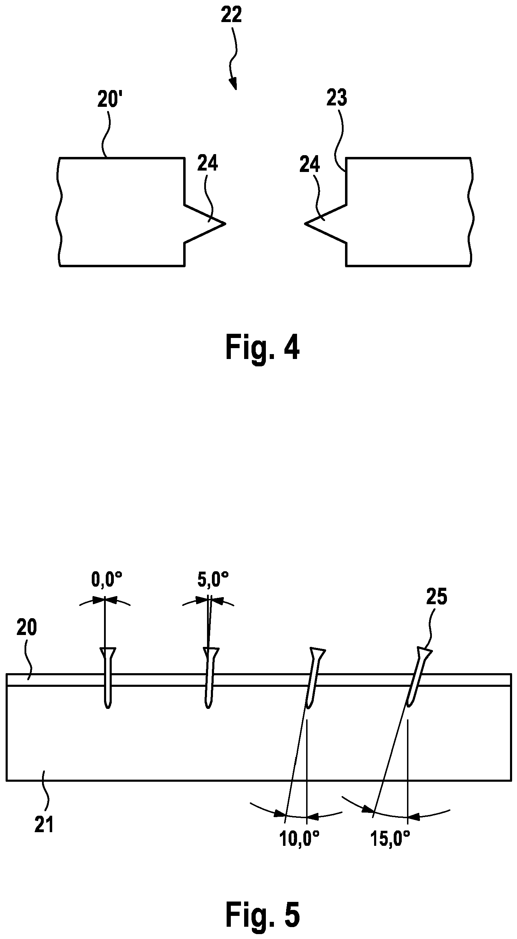

Figur 4 zeigt eine weitere Ausführungsform einer erfindungsgemäßen Knochenplatte 20' in einer seitlichen Schnittansicht. Die Knochenplatte 20' weist ein Durchgangsloch 22 auf, an dessen Innenseite 23 eine nach innen vorspringende umlaufende Lippe 24 angeordnet ist. Beim Herstellen einer Gewindeverbindung zwischen dem Kopfgewinde einer Knochenschraube und der Knochenplatte 20' wird die Lippe 24 vom Kopfgewinde der Knochenschraube umgeformt.

Dabei kann der Winkel zwischen der Knochenschraube und der Knochenplatte innerhalb gewisser Grenzen frei gewählt werden.

Figur 5 zeigt ein erfindungsgemäßes Fixationssystem mit einer Knochenplatte 20 und vier Knochenschrauben 25 in einer seitlichen Schnittansicht. Die Knochenplatte 20 liegt auf einem Knochen 21 auf. Die Knochenschrauben 25 können in frei wählbaren Winkeln zwischen 0° und 15° in die Knochenplatte 20 und in den Knochen 21 eingeschraubt werden. Die Verbindung ist also winkelvariabel. Durch die Gewindeverbindung zwischen dem Kopf der Knochenschraube 25 und der Knochenplatte 20 ist die Verbindung zudem winkelstabil, also auf einen festen Winkel zwischen der Knochenschraube 25 und der Knochenplatte 20 festgelegt.

## Patentansprüche

1. Knochenschraube zur Fixierung eines Verbindungsträgers (20) an einem Knochen (21), mit einem Schraubenkopf (13), welcher ein Kopfgewinde (15) zur Herstellung einer Gewindeverbindung mit dem Verbindungsträger (20) aufweist, mit einem Schaft (14), welcher ein Knochengewinde (16) zum Eingriff in das Knochenmaterial aufweist, und mit einem Übergangsbereich zwischen dem Schraubenkopf (13) und dem Schaft (14), wobei die Knochenschraube an ihrer Oberfläche eine harte Beschichtung (17, 18) aufweist, welche eine größere Härte als das beschichtete Material aufweist, **dadurch gekennzeichnet, dass** die Beschichtung im Bereich des Schraubenkopfs (13) eine geringere Dicke aufweist als im Bereich des Schafts (14).

2. Knochenschraube nach Anspruch 1, **dadurch gekennzeichnet, dass** die Beschichtung (17) im Bereich des Schraubenkopfes (13) eine Dicke zwischen 10 nm und 70 nm, bevorzugt zwischen 20 nm und 50 nm aufweist.

3. Knochenschraube nach einem der Ansprüche 1 oder 2, **dadurch gekennzeichnet, dass** die Beschichtung (18) im Bereich des Schafts (14) eine Dicke zwischen 80 nm und 5 µm aufweist.

4. Knochenschraube nach einem der Ansprüche 1 bis 3, **dadurch gekennzeichnet, dass** die Beschichtung (17, 18) Titanoxid umfasst.

5. Knochenschraube nach einem der Ansprüche 1 bis 4, **dadurch gekennzeichnet, dass** die Beschichtung (17, 18) aus Titanoxid besteht.

6. Knochenschraube nach einem der Ansprüche 1 bis 5, **dadurch gekennzeichnet, dass** die Knochenschraube aus Reintitan, bevorzugt aus Reintitan Grade 4 besteht.

7. Knochenschraube nach einem der Ansprüche 1 bis 5, **dadurch gekennzeichnet, dass** die Knochenschraube aus einer Titanlegierung, bevorzugt aus TiAl6V4 besteht.

8. Fixationssystem für Knochen mit einem Verbindungsträger (20), welcher wenigstens ein Durchgangsloch (22) aufweist, und mit wenigstens einer Knochenschraube nach einem der Ansprüche 1 bis 7, **dadurch gekennzeichnet, dass** die Knochenschraube in das Durchgangsloch (22) zur Herstellung einer Gewindeverbindung zwischen dem Kopfgewinde (15) und dem Verbindungsträger (20) eingesetzt wird.

9. Fixationssystem nach Anspruch 8, **dadurch gekennzeichnet, dass** der Verbindungsträger (20) im Wesentlichen aus Titan besteht.

10. Fixationssystem nach Anspruch 8 oder 9, **dadurch gekennzeichnet, dass** der Verbindungsträger im Bereich eines Durchgangsloches eine Materiallippe umfasst, welche beim Herstellen einer Gewindeverbindung vom Kopfgewinde der Knochenschraube umgeformt wird.

## Claims

1. Bone screw for fixing a connection support (20) to a bone (21), with a screw head (13), which has a head thread (15) for producing a threaded connection to the connection support (20), with a shank (14), which has a bone thread (16) for engaging in the bone substance, and with a transition area between the screw head (13) and the shank (14), the bone screw having on its surface a hard coating (17, 18) which has a greater hardness than the coated material, **characterized in that** the coating has a lesser thickness in the area of the screw head (13) than in the area of the shank (14).

2. Bone screw according to Claim 1, **characterized in that** the coating (17) in the area of the screw head (13) has a thickness of between 10 nm and 70 nm, preferably of between 20 nm and 50 nm.

3. Bone screw according to either of Claims 1 and 2, **characterized in that** the coating (18) in the area of the shank (14) has a thickness of between 80 nm and 5 µm.

4. Bone screw according to one of Claims 1 to 3, **characterized in that** the coating (17, 18) comprises titanium oxide.

5. Bone screw according to one of Claims 1 to 4, **characterized in that** the coating (17, 18) consists of titanium oxide.

6. Bone screw according to one of Claims 1 to 5, **characterized in that** the bone screw consists of pure titanium, preferably of pure titanium grade 4.

7. Bone screw according to one of Claims 1 to 5, **characterized in that** the bone screw consists of a titanium alloy, preferably of TiAl6V4.

8. Fixation system for bones, with a connection support (20) having at least one through-hole (22), and with at least one bone screw according to one of Claims 1 to 7, **characterized in that** the bone screw is inserted into the through-hole (22) in order to produce a threaded connection between the head thread (15) and the connection support (20).

9. Fixation system according to Claim 8, **characterized in that** the connection support (20) consists essentially of titanium.

10. Fixation system according to Claim 8 or 9, **characterized in that** the connection support comprises, in the area of a through-hole, a material lip which, upon production of a threaded connection, is deformed by the head thread of the bone screw.

## Revendications

1. Vis à os destinée à la fixation d'une traverse de jonction (20) sur un os (21), avec une tête de vis (13), laquelle présente un filetage de tête (15) en vue de la réalisation d'une liaison filetée avec la traverse de jonction (20), avec une tige (14), laquelle présente un filetage à os (16) destiné à une intervention dans la matière osseuse, et avec une zone de passage entre la tête de vis (13) et la tige (14);
la vis à os présentant, au niveau de sa surface, un revêtement (17, 18) dur, lequel présente une plus grande dureté que le matériau de revêtement;
**caractérisée en ce que** le revêtement présente, dans la zone de la tête de vis (13), une épaisseur plus petite que dans la zone de la tige (14).

2. Vis à os selon la revendication 1, **caractérisée en ce que** le revêtement (17) présente, dans la zone de la tête de vis (13), une épaisseur comprise entre 10 nm et 70 nm, de préférence comprise entre 20 nm et 50 nm.

3. Vis à os selon l'une des revendications 1 ou 2, **caractérisée en ce que** le revêtement (18) présente, dans la zone de la tige (14), une épaisseur comprise entre 80 nm et 5 µm.

4. Vis à os selon l'une des revendications 1 à 3, **caractérisée en ce que** le revêtement (17, 18) comprend de l'oxyde de titane.

5. Vis à os selon l'une des revendications 1 à 4, **caractérisée en ce que** le revêtement (17, 18) est constitué à partir d'oxyde de titane.

6. Vis à os selon l'une des revendications 1 à 5, **caractérisée en ce que** la vis à os est constituée à partir de titane pur, de préférence à partir de titane pur de grade 4.

7. Vis à os selon l'une des revendications 1 à 5, **caractérisée en ce que** la vis à os est constituée à partir d'un alliage en titane, de préférence à partir de TiAl6V4.

8. Système de fixation pour des os avec une traverse de jonction (20), laquelle présente tout au moins un orifice de passage (22), et avec tout au moins une vis à os selon l'une des revendications 1 à 7, **caractérisé en ce que** la vis à os est insérée dans l'orifice de passage (22) en vue de la réalisation d'une liaison filetée entre le filetage de tête (15) et la traverse de jonction (20).

9. Système de fixation selon la revendication 8, **caractérisé en ce que** la traverse de jonction (20) est constituée, pour l'essentiel, à partir de titane.

10. Système de fixation selon la revendication 8 ou 9, **caractérisé en ce que** la traverse de jonction comprend, dans la zone d'un orifice de passage, une lèvre de matière, laquelle est remodelée lors de la réalisation d'une liaison filetée du filetage de tête de la vis à os.
